# EUROPÄISCHE PATENTSCHRIFT

(11) **EP 2 685 957 B1**
(45) Veröffentlichungstag und Bekanntmachung des Hinweises auf die Patenterteilung: **25.05.2016**
(21) Anmeldenummer: 12732527.2
(22) Anmeldetag: 15.06.2012
(51) Int. Cl.: A61J 1/10, G01M 3/32, A61J 1/16, B32B 27/12, B32B 1/02, B32B 3/30, B32B 5/02

(54) **BIOREAKTORBEHÄLTER UND INTEGRITÄTSPRÜFUNGSVERFAHREN FÜR BIOREAKTORBEHÄLTER**
BIOREACTOR CONTAINER AND INTEGRITY CHECK METHOD FOR BIOREACTOR CONTAINERS
CONTENANT DE BIORÉACTEUR ET PROCÉDÉ DE CONTRÔLE DE L'INTÉGRITÉ POUR UN CONTENANT DE BIORÉACTEUR

(30) Priorität: 30.06.2011 DE 102011106162
(43) Veröffentlichungstag der Anmeldung: 22.01.2014
(60) Teilanmeldung: 14000482.1
(73) Patentinhaber: Sartorius Stedim Biotech GmbH, 37079 Göttingen (DE)
(72) Erfinder: DAHLBERG, Martin, 37120 Bovenden (DE); GAY, Isabelle, F-13124 Peypin (FR); BÖTTCHER, Lars, 34212 Melsungen (DE); OBERMANN, Stefan, 37139 Adelebsen (DE); SANDROCK, Rainer, 34134 Kassel (DE)
(74) Vertreter: Müller-Boré & Partner Patentanwälte PartG mbB
(86) Internationale Anmeldenummer: PCT/EP2012/002541
(87) Internationale Veröffentlichungsnummer: WO 2013/000544

(56) Entgegenhaltungen:
- EP-A1- 0 967 472
- WO-A1-00/22398
- WO-A1-2008/071358
- DE-U1-202012 005 987
- US-A- 2 054 204
- US-A- 2 297 375
- US-A- 3 813 923
- US-A- 5 988 422
- US-A1- 2009 022 985

## Beschreibung

Die vorliegende Beschreibung betrifft einen Bioreaktorbehälter sowie ein Verfahren zum zerstörungsfreien Prüfen der Integrität von Bioreaktorbehältern.

In der pharmazeutischen und biotechnologischen Industrie werden flexible Behälter, wie zum Beispiel Beutel, als Bioreaktorbehälter für die Prozeßführung bzw. Lagerung verwendet. Die Bioreaktorbehälter können vor der eigentlichen Verwendung durch den Herstellungsprozeß, Transport oder Handhabung beschädigt werden. Es empfiehlt sich daher, vor der eigentlichen Verwendung einen Integritätstest des Bioreaktorbehälters durchzuführen. Ein Integritätstest ist auch nach der Verwendung des Bioreaktorbehälters sinnvoll, um nachzuweisen, daß während der gesamten Prozeßführung die Integrität des Bioreaktorbehälters aufrecht erhalten wurde.
Der Begriff "Bioreaktorbehälter" im Sinne der Anmeldung umfaßt gleichermaßen Bioreaktoren sowie Behälter mit bereichsweise flexiblen Wänden, die beispielsweise zur Aufnahme, zum Mischen, zur Speicherung und zum Spenden von sterilen Medien dienen.

Gängige Testmethoden zur Integritätsprüfung des Bioreaktorbehälters sind die Druckabfallmethode, die Durchflußmessung und Spurengasanalyse unter Verwendung eines Testgases. Allen Testmethoden gemein ist, daß zwischen dem Innenraum des Bioreaktorbehälters und einer den Bioreaktorbehälter aufnehmenden Testvorrichtung bzw. der Umgebung des Bioreaktorbehälters ein Differenzdruck erzeugt wird. Dazu wird nach dem Herstellen bzw. vor und/oder nach der Benutzung der zu prüfende Bioreaktorbehälter in eine Testvörrichtung zum Prüfen der Integrität angeordnet. Dabei besteht die Gefahr, daß der zu prüfende Bioreaktorbehälter bei der dazu notwendigen Handhabung beschädigt wird, so daß in dem an sich dichten Bioreaktorbehälter ein Leck entsteht.

Die Druckschrift EP 0 967 472 A1 offenbart einen Behälter mit den im Oberbegriff des Anspruchs 1 genannten Merkmalen.

Es ist daher eine Aufgabe der Erfindung, einen Bioreaktorbehälter und ein Verfahren zum Prüfen der Integrität von Bioreaktorbehältern bereitzustellen, welcher bzw. welches eine verbesserte Integritätsprüfung des Bioreaktorbehälters erlaubt.

Die Aufgabe wird durch die Merkmale der unabhängigen Ansprüche gelöst. Bevorzugte Ausführungsformen sind Gegenstand der abhängigen Ansprüche.

### Bioreaktorbehälter gemäß einem Aspekt

Ein Aspekt der vorliegenden Erfindung betrifft einen Bioreaktorbehälter umfassend:
- eine zumindest bereichsweise flexible Wandung und
- zumindest eine Behälteröffnung,
wobei die Wandung des Bioreaktorbehälters zumindest eine fluiddichte Innenschicht aufweist und
wobei die Wandung des Bioreaktorbehälters eine zumindest bereichsweise fluiddurchlässige Außenschicht aufweist.

Vorteilhafterweise kann der Bioreaktorbehälter bezüglich seiner Integrität bzw. Dichtheit geprüft werden, wenn sich der Bioreaktorbehälter in einer Bioreaktorbehälteraufnahme einer Bioreaktorvorrichtung befindet, welche für den betriebsgemäßen Gebrauch des Bioreaktorbehälters verwendet wird. Eine weitere Handhabung des Bioreaktorbehälter und damit eine zusätzliche Gefahr der Beschädigung wird somit vorteilhafterweise vermieden.

Ein Bioreaktorbehälter umfaßt eine fluiddichte Wandung, welche zumindest bereichsweise flexibel ist. Der Begriff "Bioreaktorbehälter" im Sinne der Erfindung umfaßt gleichermaßen Bioreaktoren sowie Behälter mit bereichsweise flexiblen Wänden, die beispielsweise zur Aufnahme, zum Mischen, zur Speicherung und zum Spenden von sterilen Medien dienen. Insbesondere weist der Bioreaktorbehälter eine Wandung auf, welche aus einer Folie bzw. aus einem Verbund bzw. Laminat mehrerer Folien ausgebildet ist. Mit anderen Worten kann der Bioreaktorbehälter als Beutel ausgebildet sein. Der Bioreaktorbehälter ist volumenvariabel. Mit anderen Worten kann sich das Innenvolumen des Bioreaktorbehälters mit dem Befüllen vergrößern und mit dem Entleeren verringern. Zum Befüllen und Entleeren weist der Bioreaktorbehälter zumindest eine Behälteröffnung auf. Die zumindest eine Behälteröffnung ist bevorzugt als Anschluß bzw. Verbinder ausgebildet, so daß Fluidleitungen mittels des zumindest einen Anschlusses in einfacher Weise mit dem Bioreaktorbehälter verbindbar sind. Der Bioreaktorbehälter wird beim betriebsgemäßem Gebrauch über eine der zumindest einen Behälteröffnung mit Ausgangsstoffen bzw. Edukten befüllt und die Endstoffe bzw. Produkte werden über eine der zumindest einen Behälteröffnung entleert. Bevorzugt erfolgt das Befüllen und Entleeren des Bioreaktorbehälters über zwei verschiedene Behälteröffnungen, so daß der Bioreaktorbehälter zweckmäßigerweise zumindest zwei Behälteröffnungen aufweist. Es versteht sich, daß jede der zumindest einen Behälteröffnung fluiddicht, insbesondere steril, verschließbar bzw. verschlossen ist.

Die Dichtigkeit der Wandung gegenüber einem Fluid wird durch eine fluiddichte Innenschicht der Wandung gewährleistet. Die Innenschicht steht mit einer Innenschichtseite in Kontakt mit dem Innenvolumen des Bioreaktorbehälters. Mit anderen Worten wird die Innenschicht bei betriebsgemäßem Gebrauch durch die Edukte bzw. die Produkte der in dem Bioreaktorbehälter durchzuführenden bzw. durchgeführten biologischen, chemischen bzw. biochemischen Reaktion kontaktiert. Daher besteht die Innenschicht des Bioreaktorbehälters bevorzugt aus einem Material, welches im Hinblick auf die durchzuführende Reaktion biologisch bzw. chemisch inert ist, das heißt, daß die Innenschicht selbst im wesentlichen nicht mit den Edukten bzw. Produkten im biologischen bzw. chemischen Sinne reagiert. Bevorzugt besteht die Innenschicht aus einem Polymer, wie beispielsweise Polyethylen (PE) und/oder Polypropylen (PP). Weiter bevorzugt ist zumindest von der Innenschicht umgebene Innere des Bioreaktorbehälters sterilisierbar, beispielsweise mittels Heißdampf, Ethylenoxid-Begasung, Plasmabehandlung oder Gammabestrahlung, um die Reaktion unter sterilen Bedingungen beginnen zu können. Weiter bevorzugt sind die Behälteröffnungen als Sterilverbinder ausgebildet oder mit Sterilverbindern fluidisch verbunden, beispielsweise über einen Schlauch. In gebrauchsfertigem Zustand ist insbesondere zumindest das Innere des Bioreaktorbehälters steril. Weiter bevorzugt ist der gesamte Bioreaktorbehälter in gebrauchsfertigem Zustand vollständig sterilisiert und steril verpackt. Bevorzugt ist der Bioreaktorbehälter zum einmaligen Gebrauch bestimmt. Mit anderen Worten ist der Bioreaktorbehälter ein Einwegprodukt.

Die Innenschicht ist zumindest bereichsweise und bevorzugt vollständig flexibel ausgebildet. Es versteht sich, daß die Innenschicht der Wandung bereichsweise starr ausgebildet sein kann. Insbesondere im Bereich der zumindest einen Behälteröffnung kann die Innenschicht der Wandung im wesentlichen starr ausgebildet sein, damit die zumindest eine Behälteröffnung formstabil ist.

Die Wandung des Bioreaktorbehälters umfaßt eine zumindest bereichsweise fluiddurchlässige Außenschicht. Das heißt, das die Außenschicht zumindest bereichsweise fluiddurchlässig ist bzw. das die Wandung nur bereichsweise eine Außenschicht umfaßt. Insbesondere weist der Bereich der Wandung, welcher flexibel ausgebildet ist, eine Außenschicht auf. Demnach kann beispielsweise ein starrer Bereich der Wandung lediglich als Innenschicht ausgebildet sein. Alternativ kann die Wandung starr ausgebildet sein gleichwohl eine Außenschicht aufweisen, welche nicht fluiddurchlässig sein kann.

Die Außenschicht der Wandung ist in dem Sinne fluiddurchlässig, daß ein Fluid, welches bestimmungswidrig durch ein Leck der Innenschicht aus dem Inneren des Bioreaktorbehälters austritt, auch die Außenschicht passieren kann. Mit anderen Worten erfüllt die Außenschicht keine zusätzliche Abdichtfunktion. Vielmehr hat die Außenschicht die Funktion die Innenschicht von Gegenständen außerhalb der Bioreaktorbehälters in einer Weise zu beabstanden, daß ein solcher Gegenstand die Innenschicht im wesentlichen nicht unmittelbar mechanisch kontaktiert, sondern lediglich die Außenschicht kontaktiert, wodurch auch vorteilhafterweise die mechanische Beanspruchung der Innenschicht reduziert wird. Weiter kann ein Leck in der Innenschicht nicht mittels des Gegenstandes von außen abgedichtet werden, da die Außenschicht fluiddurchlässig ist. Ein aus dem Bioreaktorbehälter durch die Innenschicht austretendes Fluid würde demnach einen Weg durch die fluiddurchlässige Außenschicht bis zu einer Stelle der Außenschicht finden, welche nicht mit dem Gegenstand kontaktiert und dort in die Umgebung gelangen. Ein solcher Gegenstand, kann beispielsweise eine Bioreaktorbehälteraufnahme einer Bioreaktorvorrichtung sein.

Bioreaktorbehälter zur Durchführung einer biologischen Reaktion können bevorzugt ein Innenvolumen von etwa 5 Milliliter bis etwa 3000 Liter, bevorzugt etwa 2 Liter, etwa 5 Liter, etwa 10 Liter, etwa 50 Liter, etwa 100 Liter, etwa 250 Liter, etwa 500 Liter oder etwa 1000 Liter aufweisen. Üblicherweise sind Bioreaktorbehälter bei betriebsgemäßem Gebrauch mit einer wässrigen Lösung gefüllt, so daß dementsprechend der Inhalt des Bioreaktorbehälters eine Masse von etwa 5 g bis etwa 3000 kg aufweist. Da die Wandungen des Bioreaktorbehälters in der Regel nicht dem inneren Druck standhalten können, welcher durch die Masse des Inhalts des Bioreaktorbehälters erzeugt wird, sind Bioreaktorbehälter in der Regel in der Bioreaktorbehälteraufnahme der Bioreaktorvorrichtung angeordnet und darin befestigt. Dabei liegt die Wandung des Bioreaktorbehälters zumindest bereichsweise an der Wandung der Bioreaktorbehälteraufnahme an, so daß die Bioreaktorbehälteraufnahme die Wandung des Bioreaktorbehälters stützt. Die Wandungen solcher Bioreaktorbehälteraufnahmen sind üblicherweise aus glattem Edelstahl ausgebildet, um eine Verunreinigung zu erschweren und eine Reinigung zu erleichtern.

Wird nun nach dem Anordnen des Bioreaktorbehälters in der Bioreaktorbehälteraufnahme ein Integritätstest durchgeführt, wobei das Innere des Bioreaktorbehälters mit einem Fluid beaufschlagt wird, um einen Überdruck zur Umgebung zu erzeugen, so könnte die Wandung der Bioreaktorbehälteraufnahme ein gegebenenfalls vorhandenes Leck in der Wandung des Bioreaktorbehälters abdecken. Da die Außenschicht der Wandung des Bioreaktorbehälters jedoch fluiddurchlässig ist, ist es vorteilhafterweise nicht möglich, daß eine glatte Wandung der Bioreaktorbehälteraufnahme das Leck in der Wandung des Bioreaktorbehälters dadurch abdichtet, daß die Wandung des Bioreaktorbehälters durch den im Inneren des Bioreaktorbehälters herrschenden Druck gegen die Wandung der Bioreaktorbehälteraufnahme gepreßt wird. Dadurch ist vorteilhafterweise eine verbesserte Integritätsprüfung möglich, durch welche Lecks in der Wandung des Bioreaktorbehälters mit größerer Sicherheit bestimmt werden können, da vermieden wird, während der Integritätsprüfung ein Leck unbeabsichtigt zu verschließen.

Weiter vorteilhafterweise ist die Integritätsprüfung des Bioreaktorbehälters vor und/oder nach dem Versuchsablauf bzw. dem Produktionsablauf "in situ" durchführbar, also wenn der Bioreaktorbehälter in der Bioreaktorbehälteraufnahme angeordnet ist, wodurch vorteilhafterweise eine zusätzliche Handhabung des Bioreaktorbehälters entfällt und damit ein Risiko den Bioreaktorbehälter durch die zusätzliche Handhabung zu beschädigen.

Die Außenschicht ist vorzugsweise porös bzw. umfaßt ein poröses Material, wobei die einzelnen Porenvolumen zweckmäßigerweise untereinander derart verbunden sind, das die Außenschicht fluiddurchlässig ist. Weiter bevorzugt kann die fluiddurchlässige Außenschicht eine richtungsabhängige bzw. anisotrope Fluiddurchlässigkeit aufweisen. Beispielsweise kann die fluidische Leitfähigkeit der Außenschicht in einer Richtung parallel zu einer Normalen der Außenschichtoberfläche größer sein als in einer Richtung senkrecht zur dieser Normalen, also parallel zur Erstreckung der Außenschichtoberfläche.

Vorzugsweise ist die fluiddurchlässige Außenschicht mit der fluidundurchlässigen Innenschicht lösbar oder unlösbar verbunden. Die Außenschicht kann mit der Innenschicht vollflächig oder bereichsweise verbunden sein, beispielsweise durch Verkleben, Verschweißen oder Laminieren. Alternativ kann die Außenschicht auch lediglich über die Innenschicht gezogen bzw. gestülpt sein. Mit anderen Worten kann die Außenschicht lösbar an der Innenschicht angeordnet oder fest mit der Innenschicht verbunden bzw. an der Innenschicht befestigt sein. Die Verbindung zwischen der Außenschicht und der Innenschicht kann auch lediglich entlang von Verbindungslinien, Verbindungskanten oder an Verbindungsecken erfolgen.

Dementsprechend kann die Außenschicht zumindest bereichsweise von der Innenschicht beabstandet sein, wobei der Zwischenraum zwischen der Außenschicht und der Innenschicht beispielsweise mit Luft oder einem anderen Gas gefüllt sein kann.

Vorzugsweise umfaßt die fluiddurchlässige Außenschicht ein gewebtes Textil, ein nicht gewebtes Textil und/oder einen Schaumstoff. Als bevorzugte Materialien für die Außenschicht können Vliesstoffe, wie beispielsweise Spinnvliese aus Polypropylen verwendet werden. Ein solches Vlies ist zum Beispiel Novatexx 2019 Viledon von der Firma Freudenberg Filtratation Technologies KG aus Polypropylen mit einem Gewicht von 17-100 g/m² und einer Luftdurchlässigkeit von 1000-5000 I/m²s bei einer Druckdifferenz von 1 bar mit einer Materialdicke von 0,25-0,75 mm. Ein weiteres beispielhaftes Material ist unter den Handelsnamen Porex Porous Plastics XS XS49020-XS49100 von der Firma Porex Technologies GmbH erhältlich. Das Material besteht aus Polypropylen und Polyethylen mit einer Materialdicke von etwa 1,5 mm bis etwa 5 mm, bevorzugt größer als etwa 3 mm. Die Größe der Poren liegt im Bereich von etwa 20 µm bis etwa 175 µm, bevorzugt kleiner als etwa 120µm. Die Luftdurchlässigkeit liegt bei etwa 150 bis etwa 300 l/cm²min bei 1,2 inch Wassersäule. Es hat sich als vorteilhaft erwiesen, für die fluiddurchlässige Außenschicht ein Polymermaterial zu verwenden, welches wärmeleitende Zusätze, wie beispielsweise Bomitrat, enthält. Dadurch läßt sich eine Temperierung des Bioreaktorbehälters vorteilhafterweise verbessern.

Bevorzugt kann die Innenschicht mehrere Innenschichtlagen aufweisen, welche miteinander verbunden sind. Beispielsweise können zwei oder mehr Innenschichtlagen durch Laminieren miteinander verbunden sein. Dabei ist zumindest eine der Innenschichtlagen fluiddicht. Bevorzugt sind alle Innenschichtlagen fluiddicht ausgebildet. Zumindest eine - bevorzugt jede - der Innenschichtlagen ist als Sterilbarriere ausgebildet. Mit anderen Worten können Mikroorganismen, Bakterien, Viren, Prionen usw. die Innenschicht bzw. die Innenschichtlage(n) nicht durchdringen, solange die Sterilbarriere intakt ist bzw. sind.

Der Begriff "flexibel" umfaßt im Sinne der Anmeldung eine plastische sowie eine elastische Verformbarkeit. Der Begriff "Fluid" umfaßt sowohl eine gasförmige Phase, eine flüssige Phase als auch ein Gemisch aus flüssiger und gasförmiger Phase eines Stoffes.

### Bioreaktorbehälter gemäß einem Beispiel

Ein Beispiel betrifft einen Bioreaktorbehälter umfassend:
- eine zumindest bereichsweise flexible Wandung und
- zumindest eine Behälteröffnung,
wobei die Wandung des Bioreaktorbehälters eine fluiddichte Innenschicht aufweist und
wobei die Wandung des Bioreaktorbehälters eine zumindest bereichsweise strukturierte Außenschicht aufweist.

Der Bioreaktorbehälter entspricht in seinen Eigenschaften im wesentlichen dem oben beschriebenen Bioreaktorbehälter, wobei die fluiddurchlässige Außenschicht durch eine strukturierte Außenschicht ersetzt ist, welche fluiddurchlässig oder fluidundurchlässig sein kann. Daher gelten die obigen Ausführungen bezüglich des Bioreaktorbehälters, insbesondere bezüglich der Wandung, der Behälteröffnung und der Innenschicht entsprechend.

Eine strukturierte Außenschicht im Sinne der Anmeldung bedeutet, daß zumindest die Außenoberfläche der Außenschicht nicht glatt ist, sondern eine Struktur bzw. ein Relief aufweist. Insbesondere kann die Struktur durch Erhebungen und Vertiefungen ausgebildet werden, welche durch einen Wechsel der Materialstärke der Außenschicht erzeugt werden. Das heißt, die Außenschicht weist im Bereich von Erhebungen eine größere Materialstärke auf als im Bereich von Vertiefungen. Alternativ kann die Materialstärke der Außenschicht im wesentlichen konstant sein, wobei die Struktur in das Material geformt ist. Dabei ist insbesondere der flexibel ausgebildete Bereich der Wandung mit einer strukturierten Außenschicht versehen. Bevorzugt können aber auch starre Bereiche der Wandung einer strukturierte Außenschicht bzw. eine strukturierte Außenoberfläche aufweisen. Die Außenoberfläche bezieht sich auf die Fläche der Wandung, welche sich gegenüberliegend bzw. entgegengesetzt zu der dem Inneren des Bioreaktorbehälters zugewandten Seite der Wandung befindet. Typischerweise wird der Bioreaktorbehälter an der Außenoberfläche angefaßt und gehandhabt.

Im Gegensatz zu der oben beschriebenen Alternative kann die Außenschicht der Wandung fluidundurchlässig sein, wodurch die Außenschicht vorteilhafterweise eine zusätzliche Abdichtfunktion erfüllen kann. Dennoch führt die strukturierte Außenschicht zu einem äquivalenten technischen Effekt wie eine fluiddurchlässige Außenschicht in dem Sinne, daß ein Leck in der Innenschicht nicht mittels eines Gegenstandes von außen abgedichtet werden kann. Aufgrund der Struktur der Außenschicht kann ein Gegenstand in der Regel nicht mit der Außenschicht derart fluiddicht abschließen, daß ein aus dem Bioreaktorbehälter durch die Innenschicht austretendes Fluid nicht in die Umgebung gelangen würde. Insbesondere kann ein solches Abdichten nicht mittels einer Bioreaktorbehälteraufnahme einer Bioreaktorvorrichtung erfolgen. Demzufolge kann ein Integritätstest durch Beaufschlagen des Bioreaktorbehälter mit einem Überdruck erfolgen, wobei aufgrund des Lecks ein Druckabfall oder ein Fluidfluß feststellbar ist, welcher auf ein Leck schließen läßt.

Wie oben bereits beschrieben kann der Bioreaktorbehälter zur Durchführung einer biologischen Reaktion in der Bioreaktorbehälteraufnahme der Bioreaktorvorrichtung angeordnet sein, wobei die Wandung des Bioreaktorbehälters zumindest bereichsweise an der im wesentlichen glatten Wandung der Bioreaktorbehälteraufnahme anliegt.

Bei der Durchführung eines Integritätstest des Bioreaktorbehälters in der Bioreaktorbehälteraufnahme wird die strukturierte Außenschicht gegen Wandung der Bioreaktorbehälteraufnahme gepreßt. Die Struktur der Außenschicht führt dazu, daß durch das Anordnen der Außenschicht an die Bioreaktorbehälteraufnahme fluidleitende Kanäle zwischen beiden ausgebildet werden. Dadurch kann ein durch ein Leck die Außenschicht dringendes Fluid über die fluidleitenden Kanäle in die Umgebung geleitet werden, so daß das Leck in der Wandung des Bioreaktorbehälters nicht durch die Wandung der Bioreaktorbehälteraufnahme abdichtbar ist. Wie oben bereits beschrieben ist dadurch vorteilhafterweise eine verbesserte Integritätsprüfung möglich, durch welche Lecks in der Wandung des Bioreaktorbehälters mit größerer Sicherheit bestimmt werden können, da vermieden wird während der Integritätsprüfung ein Leck unbeabsichtigt zu verschließen, wobei weiter vorteilhafterweise die Integritätsprüfung des Bioreaktorbehälters vor und/oder nach dem Versuchsablauf bzw. dem Produktionsablauf "in situ" durchführbar ist.

Vorzugsweise weist die strukturierte Außenoberfläche Vertiefungen auf, welche zumindest etwa 100 µm oder etwa 200 µm tief sind. Dabei definieren bzw. bilden zwei benachbarte Vertiefungen eine Erhebung. Ebenso definieren bzw. bilden zumindest zwei benachbarte Erhebungen eine Vertiefung. Weiter bevorzugt weisen die Vertiefungen gegenüber zu den benachbarten Erhebungen zumindest eine Tiefe von größer als etwa 150 µm, weiter bevorzugt von größer als etwa 250 µm und insbesondere von größer als etwa 500 µm auf. Dadurch wird das Ableiten des aus einem Leck austretenden Fluids in die Umgebung sichergestellt.

Vorzugsweise weist die strukturierte Außenseite Erhebungen auf, welche höchstens 200 µm breit sind. Weiter bevorzugt weisen die Erhebungen eine Breite von weniger als etwa 150 µm, weiter bevorzugt von weniger als etwa 100 µm und insbesondere von weniger als etwa 50 µm auf. Dadurch wird verhindert, daß eine Erhebung paßgenau auf einem Leck positionierbar ist und dadurch das Leck durch eine einzige Erhebung abgedichtet wird. Der erwartete Durchmesser eines Lecks beträgt etwa 5 µm bis etwa 1000 µm.

Vorzugsweise sind die Erhebungen und/oder Vertiefungen der strukturierten Außenseite entlang einer Vorzugsrichtung V orientiert. Insbesondere erstrecken sich die Erhebungen und/oder Vertiefungen im wesentlichen entlang einer Längsrichtung L, wobei benachbarte Erhebungen und/oder Vertiefungen parallel zueinander orientiert sind. Die Vorzugsrichtung V entspricht dabei der Längsrichtung L entlang welcher sich die Erhebungen bzw. Vertiefungen erstrecken.

Mit anderen Worten können die Erhebungen und Vertiefungen insbesondere eine Rillenstruktur oder eine Rautenstruktur auf der Außenschicht bzw. der Außenoberfläche ausbilden.

Vorzugsweise ist die strukturierte Außenschicht mit der fluidundurchlässigen Innenschicht lösbar oder unlösbar verbunden. Die strukturierte Außenschicht kann mit der Innenschicht vollflächig oder bereichsweise verbunden sein, beispielsweise durch Verkleben, Verschweißen oder Laminieren. Alternativ kann die strukturierte Außenschicht auch lediglich über die Innenschicht gezogen bzw. gestülpt sein. Mit anderen Worten kann die strukturierte Außenschicht lösbar an der Innenschicht angeordnet oder fest mit der Innenschicht verbunden bzw. an der Innenschicht befestigt sein. Die Verbindung zwischen der strukturierte Außenschicht und der Innenschicht kann auch lediglich entlang von Verbindungslinien, Verbindungskanten oder an Verbindungsecken erfolgen. Dementsprechend kann die strukturierte Außenschicht zumindest bereichsweise von der Innenschicht beabstandet sein, wobei der Zwischenraum zwischen der strukturierte Außenschicht und der Innenschicht beispielsweise mit Luft oder einem anderen Gas gefüllt sein kann. Weiter vorzugsweise ist die strukturierte Außenschicht als sterile Barriere ausgebildet.

Vorzugsweise sind die beiden oben beschrieben alternativen Bioreaktorbehälter derart ausgebildet, daß die zumindest eine Behälteröffnung des Bioreaktorbehälter in einem ersten Zustand durch die Außenschicht umhüllt ist, wobei die zumindest eine Behälteröffnung in einem zweiten Zustand frei zugänglich ist und wobei der Bioreaktorbehälter durch ein bereichsweises Entfernen der Außenschicht von dem ersten Zustand in den zweiten Zustand überführbar ist. Die Außenschicht kann dabei fluiddurchlässig und/oder strukturiert sein.

Vorteilhafterweise kann die Außenschicht auch als Schutz und/oder steriler Verschluß bzw. Verpackung der zumindest einen Behälteröffnung dienen. Insbesondere kann die Außenschicht im ersten Zustand eine geschlossene Hülle um die weiteren Elemente des Bioreaktorbehälters ausbilden, welche bevorzugt diese Elemente steril verpackt. In der Handhabung kann der Bioreaktorbehälter in der Bioreaktorbehälteraufnahme angeordnet werden, um anschließend in den zweiten Zustand überführt zu werden, beispielsweise durch Aufreißen der Außenschicht, welche dann vorzugsweise über einen Bereich der Bioreaktorbehälteraufnahme gestülpt werden kann.

Bevorzugt kann die Außenschicht aus Polyethylen, aus einem Verbund bzw. Laminat von Polyethylen und Polypropylen oder aus einem Material, welches unter der Handelsbezeichnung Tyvek® bekannt ist, ausgebildet sein. Insbesondere Tyvek® ist heißdampfdurchlässig, so daß eine Sterilisierung des Bioreaktorbehälters mittels Heißdampf erfolgen kann, wobei dieses Material eine sterile Barriere darstellt.

### Verfahren zum Prüfen der Integrität eines Bioreaktorbehälters gemäß einem Aspekt

Ein Aspekt betrifft ein Verfahren zum Prüfen der Integrität eines Bioreaktorbehälters mit den Schritten:
- Bereitstellen eines erfindungsgemäßen Bioreaktorbehälters;
- Bereitstellen einer Bioreaktorvorrichtung mit einer Bioreaktorbehälteraufnahme;
- Anordnen des Bioreaktorbehälters zumindest teilweise in der Bioreaktorbehälteraufnahme;
- Verbinden der zumindest einen Behälteröffnung des Bioreaktorbehälters mit einer Fluidquelle;
- Füllen des Bioreaktorbehälters mit einem Fluid aus der Fluidquelle, um zu einem ersten Zeitpunkt T₁ einen vorbestimmten Überdruck P₁ in dem Bioreaktorbehälter zu erzeugen, wobei sich die Außenschicht des Bioreaktorbehälters zumindest bereichsweise an eine Innenwandung der Bioreaktorbehälteraufnahme anlegt;
- Diskriminieren, ob der Bioreaktorbehälter hinreichend dicht ist.

Dabei kann das Diskriminieren insbesondere anhand einer Druckdifferenz (P₂-P₁) zwischen einem Überdruck P₂ zu einem späteren Zeitpunkt T₂ und dem bestimmten Überdruck zu Zeitpunkt T₁ erfolgen. Alternativ oder zusätzlich kann das Diskriminieren anhand einer Fluidmenge M erfolgen, welche nach dem Zeitpunkt T₁ dem Bioreaktorbehälter zugeführt wurde, um den Überdruck P₁ konstant zu halten. Weiter alternativ oder zusätzlich kann das Diskriminieren anhand einer Detektion von Fluidpartikeln außerhalb des Bioreaktorbehälters erfolgen, welche dem Bioreaktorbehätter zugeführt wurden. Vorteilhafterweise kann die Genauigkeit des Diskriminierens bzw. des Bestimmens und Entscheidens, ob der Bioreaktorbehälter dicht ist, erhöht werden, wenn zwei oder drei der oben beschriebenen Größen erfaßt werden. Die Wahrscheinlichkeit, daß die Dichtigkeit des Bioreaktorbehälters falsch klassifiziert bzw. bestimmt wird, sinkt dadurch vorteilhafterweise.

Ein Integritätstest gibt Auskunft über die Integrität bzw. Dichtigkeit des Bioreaktorbehälters, insbesondere ob Fluide aus einem Leck austreten oder eindringen können. Insbesondere ist die Integrität verletzt und der Bioreaktorbehälter unbrauchbar, wenn Mikroorganismen durch ein Leck in das Innere des Bioreaktorbehälters eindringen können, wodurch die Reaktion innerhalb des Bioreaktorbehälters beeinträchtigt und die entstandenen Produkte unbrauchbar sind. Vorteilhafterweise kann der Bioreaktorbehälter an der Innenwandung der Bioreaktorbehälteraufnahme anliegen, wobei das Ergebnis des Integritätstests nicht beeinflußt wird, da das Abdichten von vorhandenen Lecks durch die Innenwandung vermieden wird. Insbesondere kann ein Integritätstest durchgeführt werden, wenn der Bioreaktorbehälter bereits in der Bioreaktorbehälteraufnahme der Bioreaktorvorrichtung aufgenommen ist, mit welcher später die eigentliche Reaktion durchgeführt wird.

Das Diskriminieren, ob der Behälter hinreichend dicht ist kann insbesondere mittels der Druckabfallmethode, durch Messung des Fluidzuflusses bei konstantem Druck und mittels einem Testgas als Fluid erfolgen. Das Testgas ist zweckmäßigerweise ein Gas welches nicht oder nur in Spuren in der Atmosphäre vorkommt und deshalb leicht an den Leckstellen mittels eines Gasdetektors detektieren werden kann. Der Überdruck P1 des Fluids innerhalb des Bioreaktorbehälters gegenüber dem Atmosphärendruck beträgt zwischen etwa 20 mbar und etwa 500 mbar, bevorzugt zwischen etwa 50 mbar und etwa 300 mbar.

Vorzugsweise umfaßt das Verfahren die anschließenden Schritte:
- Füllen des Bioreaktorbehälters mit Edukten;
- Durchführen einer chemischen bzw. biochemischen Reaktion in dem Bioreaktorbehälter;
- Ablassen des Inhalts aus dem Bioreaktorbehälter.

Vorteilhafterweise kann der Integritätstest in das Produktionsverfahren integriert werden, da direkt im Anschluß an den Integritätstest der Bioreaktorbehälter mit den Edukten bzw. Ausgangsstoffen der Reaktion befüllt werden kann. Um das restliche für den Integritätstest verwendete Fluid aus dem Bioreaktorbehälter zu entfernen, können die Ausgangsstoffe zweckmäßigerweise über eine am Bioreaktorbehälter unten gelegene Behälteröffnung zugeführt werden, während das Fluid über eine oben gelegene Behälteröffnung entlüftet wird, insbesondere über einen Sterilfilter. Insbesondere bei proteinhaltigen Ausgangsstoffen wird ein Aufschäumen vorteilhafterweise vermieden.

Vorzugsweise erfolgt das Diskriminieren, ob der Bioreaktorbehälter hinreichend dicht ist, vor und/oder nach dem Durchführen der biochemischen Reaktion in dem Bioreaktorbehälter. Bevorzugt umfaßt das Verfahren die an das Ablassen anschließenden Schritte:
- Verbinden der zumindest einen Behälteröffnung des Bioreaktorbehälters mit einer Fluidquelle;
- Füllen des Bioreaktorbehälters mit einem Fluid aus der Fluidquelle, um zu einem ersten Zeitpunkt T₁ einen vorbestimmten Überdruck P₁ in dem Bioreaktorbehälter zu erzeugen, wobei sich die Außenschicht des Bioreaktorbehälters zumindest bereichsweise an eine Innenwandung der Bioreaktorbehälteraufnahme anliegt;
- Diskriminieren, ob der Behälter hinreichend dicht ist.

Das Diskriminieren erfolgt bevorzugt anhand einer Druckdifferenz (P₂-P₁) zwischen einem Überdruck P₂ zu einem späteren Zeitpunkt T₂ und dem bestimmten Überdruck zu Zeitpunkt T₁. Alternativ erfolgt das Diskriminieren anhand einer Fluidmenge M, welche nach dem Zeitpunkt T₁ dem Bioreaktorbehälter zugeführt wurde, um den Überdruck P₁ konstant zu halten. Weiter alternativ kann das Diskriminieren anhand einer Detektion von Fluidpartikeln außerhalb des Bioreaktorbehälters erfolgen, wobei die Fluidpartikel (z.B. ein Testgas) dem Bioreaktorbehälter zugeführt wurden.

Mit anderen Worten kann vorteilhafterweise nach Beendigung der Reaktion ein abschließender Integritätstest durchgeführt werden, um zu überprüfen, ob der Bioreaktorbehälter über den gesamten Zeitraum der Reaktion seine Integrität behalten hat.

### Figurenbeschreibung

Nachfolgend werden bevorzugte Ausführungsformen der vorliegenden Erfindung anhand der beigefügten Zeichnungen beispielhaft erläutert. Einzelne Merkmale der gezeigten bevorzugten Ausführungsformen können zu weiteren bevorzugten Ausführungsformen kombiniert werden. Es zeigen:
- Figur 1: eine perspektivische Ansicht einer bevorzugten Ausführungsform eines Bioreaktorbehälters;
- Figur 2: eine perspektivische Ansicht einer weiteren bevorzugten Ausführungsform eines Bioreaktorbehälters;
- Figur 3: eine perspektivische Ansicht einer weiteren bevorzugten Ausführungsform eines Bioreaktorbehälters;
- Figur 4: einen Schnitt durch eine Ausführungsform einer Wandung mit einer fluiddichten Innenschicht und einer fluiddurchlässigen Außenschicht;
- Figur 5a: einen Schnitt durch eine beispielhafte strukturierte Außenschicht;
- Figur 5b: eine Draufsicht auf die strukturierte Außenschicht;
- Figur 6a: einen Schnitt durch eine weitere beispielhafte strukturierte Außenschicht;
- Figur 6b: eine Draufsicht auf die strukturierte Außenschicht;

- Figur 7a: einen Schnitt durch eine weitere beispielhafte strukturierten Außenschicht;
- Figur 7b: eine Draufsicht auf die strukturierte Außenschicht;
- Figur 8: eine schematische Ansicht einer bevorzugten Ausführungsform eines Bioreaktorbehälters;
- Figur 9: eine schematische Ansicht einer Vorrichtung zum Prüfen der Integrität des Bioreaktorbehälters;
- Figur 10: eine bevorzugte Ausführungsform eines Bioreaktorbehälters.

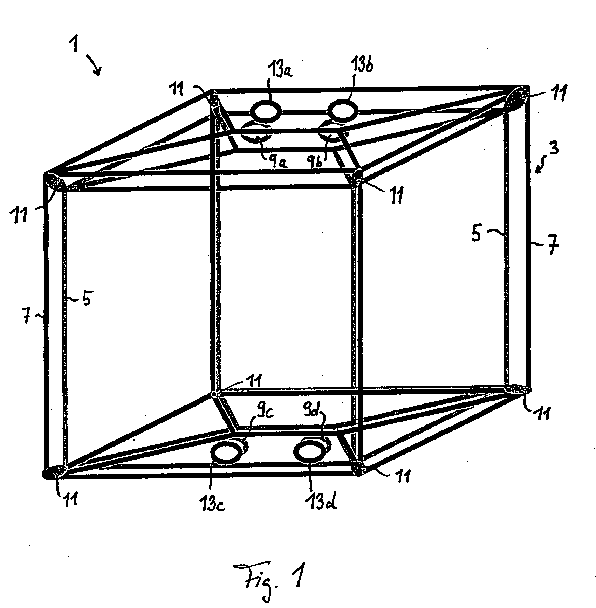

Die **Figur 1** zeigt eine bevorzugte Ausführungsform eines Bioreaktorbehälters 1 in einer perspektivischen Ansicht. Diesbezüglich sein angemerkt, daß der Begriff "Bioreaktorbehälter" im Sinne der Erfindung gleichermaßen Bioreaktoren sowie Behälter mit bereichsweise flexiblen Wänden umfaßt, die beispielsweise zur Aufnahme, zum Mischen, zur Speicherung und zum Spenden von sterilen Medien dienen. Der Bioreaktorbehälter 1 umfaßt eine Wandung 3, welche eine fluidundurchlässige bzw. fluiddichte Innenschicht 5 und eine fluiddurchlässige Außenschicht 7 aufweist. Die Innenschicht 5 ist aus einem flexiblen, nicht porösen Material ausgebildet, so daß durch die Innenschicht ein variables Innenvolumen umgeben ist. Das Innenvolumen des Bioreaktorbehälters 1 ist über die vier Behälteröffnungen 9a, 9b, 9c, 9d mit der Umgebung oder mit weiteren Elementen, wie zum Beispiel Fluidleitungen, fluidisch verbindbar. Es versteht sich, daß die flexible Innenschicht 5 im Bereich der Behälteröffnungen versteift bzw. starr ausgebildet sein kann, damit die Behälteröffnungen 9a, 9b, 9c, 9d formstabil sind und gegebenenfalls daran anschließende Anschlüsse und Verbinder dicht bleiben. Über die Behälteröffnungen 9a, 9b, 9c, 9d kann der Bioreaktorbehälter 1 befüllt und entleert werden. Die Außenschicht 7 ist aus einem porösen, fluiddurchlässigen Material ausgebildet, welches im wesentlichen beabstandet von der Innenschicht 5 angeordnet ist. Die Innenschicht 5 und die Außenschicht 7 sind dabei punktuell im Bereich der Ecken miteinander verbunden.

Der in der Figur 1 gezeigte Bioreaktorbehälter 1 weist eine quaderförmige oder würfelförmige Gestalt auf. Daher sind die Innenschicht 5 und die Außenschicht 7 an den acht Ecken des Quaders bzw. des Würfels miteinander verbunden. Diese Verbindung kann beispielsweise durch Verschweißen bzw. Verkleben erfolgen, insbesondere an Laschen 11, welche von der Innenschicht 5 und/oder der Außenschicht 7 überstehen. Alternativ oder zusätzlich können die Innenschicht 5 und die Außenschicht 7 im Bereich der Behälteröffnungen 9a, 9b, 9c, 9d miteinander verbunden, verschweißt bzw. verklebt sein.

Es versteht sich, daß der Bioreaktorbehälter 1 auch eine tetraederförmige, eine zylindrische, eine kugelförmige, eine prismenförmige oder eine sonstige beliebige Gestalt aufweisen kann. Die Zahl der Verbindungsbereiche, an denen die Innenschicht 5 und die Außenschicht 7 dann miteinander verbunden sind, kann dann entsprechend variieren.

Die Außenschicht 7 kann unabhängig von der äußeren Gestalt starr oder flexibel ausgebildet sein. Vorteilhafterweise ermöglicht eine starre Außenschicht 7, daß der Bioreaktorbehälter stets ein definiertes Volumen einnimmt und mittels der starren Außenschicht 7 handhabbar ist, ohne während der Handhabung einen Druck auf die Innenschicht auszuüben. Alternativ kann die Außenschicht 7 flexibel ausgebildet sein, wodurch sich der Bioreaktorbehälter 1 in einfacher Weise zusammenlegen und platzsparend lagern läßt. Im Bereich der Behälteröffnungen 9a, 9b, 9c, 9d weist die Außenschicht 7 bevorzugt Durchtritte 13a, 13b, 13c, 13d auf, durch welche die Behälteröffnungen 9a, 9b, 9c, 9d zugänglich sind, oder durch welche ausgehend von den Behälteröffnungen 9a, 9b, 9c, 9d Verbinder bzw. Leitungen hindurchragen können.

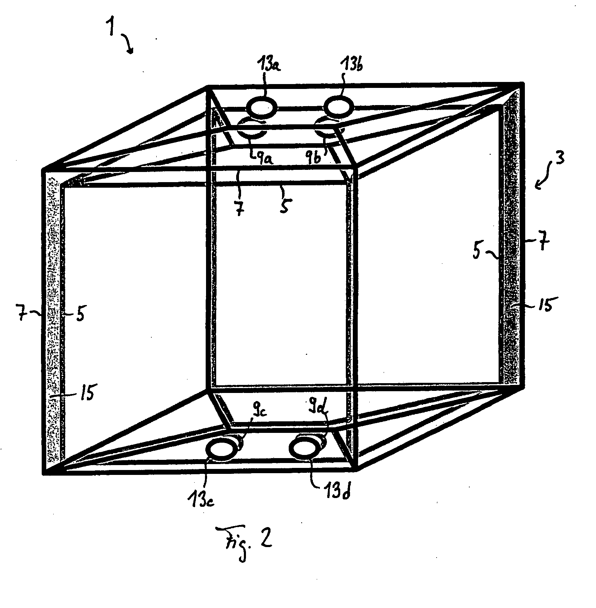

Die **Figur 2** zeigt eine weitere bevorzugte Ausführungsform eines Bioreaktorbehälters 1 in einer perspektivischen Ansicht. Der Aufbau des Bioreaktorbehälter 1 entspricht im wesentlichen dem Aufbau des in der Figur 1 gezeigten Bioreaktorbehälters 1, wobei zu Figur 1 identische Elemente in der Figur 2 mit den gleichen Bezugszeichen gekennzeichnet sind.

Der in der Figur 2 gezeigte Bioreaktorbehälter 1 weist eine Innenschicht 5 und eine Außenschicht 7 auf, welchen entlang von Schweißnähten 13 miteinander verbunden sind. Zweckmäßigerweise sind die Innenschicht 5 und die Außenschicht 7 an den Kanten der Wandung 3 miteinander verbunden, beispielsweise durch Verschweißen bzw. Verkleben. Die linienförmige Verbindung der Innenschicht 5 mit der Außenschicht 7 führt vorteilhafterweise zu einer stabileren Verbindung, welche eine erhöhte Verwindungssteifigkeit des Bioreaktorbehälters 1 bewirkt.

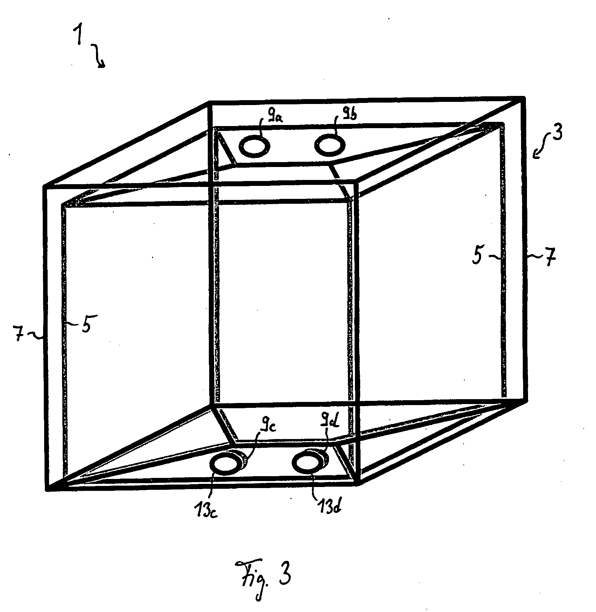

Die **Figur 3** zeigt eine weitere bevorzugte Ausführungsform eines Bioreaktorbehälters 1 in einer perspektivischen Ansicht. Der Aufbau des Bioreaktorbehälter 1 entspricht im wesentlichen dem Aufbau der in den Figuren 1 und 2 gezeigten Bioreaktorbehälter 1, so daß identische Elemente in der Figur 3 mit den gleichen Bezugszeichen gekennzeichnet sind.

Der in der Figur 3 gezeigte Bioreaktorbehälter 1 weist eine Innenschicht 5 und eine Außenschicht 7, wobei die Außenschicht 7 die Innenschicht 5 nur bereichsweise umschließt bzw. nur bereichsweise an der Innenschicht 5 angeordnet ist. Insbesondere weist der Bioreaktorbehälter 1 an der Seite, an welcher die Behälteröffnungen 9a, 9b ausgebildet sind, keine Außenschicht 7 auf. Dies ist insbesondere dann nicht notwendig, wenn der Bioreaktorbehälter 1 bei betriebsgemäßem Gebrauch in diesem Bereich keine Bioreaktorbehälteraufnahme mechanisch kontaktiert. Die Innenschicht 5 und die Außenschicht 7 können vollflächig aneinander anliegen bzw. vollflächig miteinander verbunden sein. Beispielsweise können die Innenschicht 5 und die Außenschicht 7 miteinander verklebt oder laminiert sein. Es versteht sich, daß die Außenschicht 7 auch lediglich lösbar über die Innenschicht 5 gestülpt sein kann. Insbesondere kann die Außenschicht 7 als ein oder mehrere separate(s) Element(e) getrennt vom restlichen Bioreaktorbehälter 1 geliefert werden, so daß die Außenschicht 7 erst bei bestimmungsgemäßem Gebrauch des Behälters an der Innenschicht 5 angeordnet wird. Zweckmäßigerweise werden die Außenschicht 7 und der restliche Bioreaktorbehälter als Set innerhalb einer Verpackungseinheit geliefert.

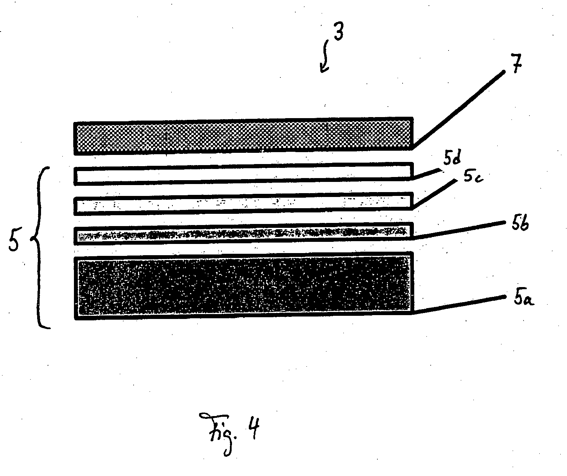

Die **Figur 4** zeigt einen Schnitt durch eine Ausführungsform einer Wandung 3 mit einer fluiddichten Innenschicht 5 und einer fluiddurchlässigen Außenschicht 7. Die Außenschicht besteht dabei in dieser bevorzugten Ausführungsform aus einer porösen Lage eines Vliesstoffes bzw. eines Schaumstoffes mit durchgängigen Poren. Vorteilhafterweise ist die Außenschicht 7 mechanisch strapazierfähig und kann daher die darunterliegende Innenschicht 5 vor mechanischen Einwirkungen schützen.

Die Innenschicht 5 umfaßt in der gezeigten bevorzugten Ausführungsform mehrere Innenschichtlagen 5a, 5b, 5c, 5d, welche miteinander verbunden sind. Beispielsweise können die Innenschichtlagen 5a, 5b, 5c, 5d durch Laminieren oder Kleben miteinander verbunden sein.

Eine Kontaktinnenschichtlage 5a besteht aus einem Material, welches im Hinblick auf die in Inneren des Bioreaktorbehälters durchzuführenden Reaktionen biologisch bzw. chemisch inert ist, das heißt, daß die Innenschicht selbst im wesentlichen nicht biologisch bzw. chemisch reagiert. Bevorzugt besteht die Kontaktinnenschichtlage 5a aus einem Polymer, wie beispielsweise Polyethylen (PE) und/oder Polypropylen (PP). Um die Wandung 3 fluiddicht im Sinne von gasdicht auszubilden, kann die Innenschicht eine gasdichte Innenschichtlage 5b bzw. eine Gasbarriere 5b aufweisen. Die mechanische Stabilität der Wandung kann durch eine oder mehrere mechanisch tragende Innenschichtlagen 5c, 5d erzeugt werden. Diese mechanisch tragenden Innenschichtlagen 5c, 5d können fluiddicht ausgebildet sein, können aber auch fluiddurchlässig bzw. gasdurchlässig sein, wenn die Kontaktinnenschichtlage 5a und die Gasbarriere 5b eine hinreichende Fluiddichtigkeit gewährleisten.

Als Alternative zu der fluiddurchlässigen bzw. porösen Außenschicht 7 kann auch eine strukturierte Außenschicht 7 in der Wandung 3 vorgesehen sein.

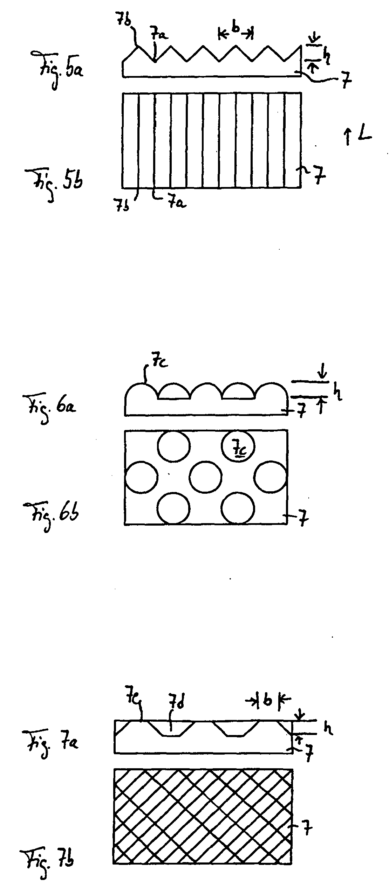

Die **Figur 5a** zeigt einen Schnitt durch und die **Figur 5 b** eine Draufsicht auf eine beispielhafte strukturierten Außenschicht 7. Die Außenschicht 7 weist in dieser Ausführungsform Vertiefungen 7a und Erhebungen 7b auf, welche sich parallel zueinander entlang einer Längsrichtung L erstrecken. Mit anderen Worten bilden die Erhebungen 7b und Vertiefungen 7a eine Rillenstruktur auf der Außenschicht 7 bzw. der Außenoberfläche aus. Die Erhebungen 7b und Vertiefungen 7a werden durch einen Wechsel der Materialstärke der Außenschicht 7 ausgebildet. Vorzugsweise umfaßt die strukturierte Außenseite Erhebungen, welche eine Breite b von höchstens etwa 200 µm, bevorzugt etwa 50 µm, und eine Höhe h von höchstens etwa 200 µm, bevorzugt etwa 50 µm aufweisen.

Die Figur 6a zeigt einen Schnitt durch und die **Figur 6 b** eine Draufsicht auf eine beispielhafte strukturierten Außenschicht 7. Die Außenschicht 7 weist in dieser Ausführungsform halbkugelförmige Erhebungen 7c bzw. Noppen 7c auf. Die Noppen 7c sind vorzugsweise regelmäßig auf der Außenschicht 7 angeordnet. Die Erhebungen bzw. Noppen 7c weisen bevorzugt eine Höhe h von höchstens etwa 200 µm auf. Die Punktauflage sollte kleiner als 5 µm, bevorzugt kleiner als 2,5 µm sein, zumindest aber höchstens den halben Durchmesser der zu detektierenden Lochgröße sein.

Die **Figur 7a** zeigt einen Schnitt durch und die **Figur 7b** ine Draufsicht auf eine beispielhafte strukturierten Außenschicht 7. Die Außenschicht 7 weist in dieser Ausführungsform Vertiefungen 7d und Erhebungen 7e auf, welche eine Rautenstruktur auf der Außenschicht 7 bzw. der Außenoberfläche ausbilden. Die Erhebungen 7e und Vertiefungen 7d werden durch einen Wechsel der Materialstärke der Außenschicht 7 ausgebildet, wobei die Erhebungen eine Breite b von höchstens etwa 5 µm, bevorzugt etwa 2,5 µm, und eine Höhe h von zumindest etwa 100 µm aufweisen.

Es versteht sich, das die Erhebungen und Vertiefungen der Außenschicht 7 kein regelmäßiges bzw. periodisches Muster ausbilden müssen, sondern eine ungeordnete, unregelmäßige Struktur ausbilden können.

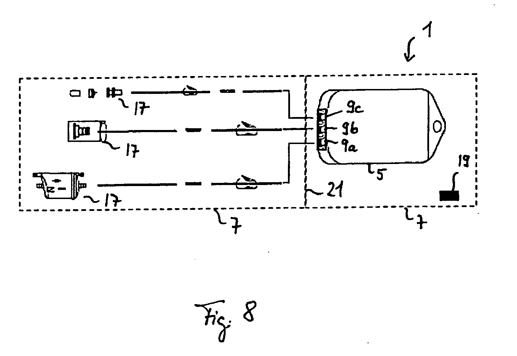

Die **Figur 8** zeigt eine schematische Ansicht einer bevorzugten Ausführungsform eines Bioreaktorbehälters 1. Die Figur 8 zeigt den Bioreaktorbehälter 1 in einem ersten Zustand, wobei die Behälteröffnungen 9a, 9b, 9c in der Innenschicht 5 des Bioreaktorbehälters 1 durch die Außenschicht 7 umhüllt sind. Mit anderen Worten bildet die Außenschicht 7 im ersten Zustand eine Außenverpackung des Bioreaktorbehälters 1, welcher den Bioreaktorbehälter und insbesondere die Behälteröffnungen 9a, 9b, 9c und gegebenenfalls mit den Behälteröffnungen 9a, 9b, 9c verbundene Komponenten 17, wie beispielsweise Fluidanschlüsse, Verbinder, Sterilverbinder, bzw. Sterilfilter umgibt, insbesondere steril umgibt. Vorteilhafterweise dient die Außenschicht 7 so als Schutz und/oder steriler Verschluß bzw. Verpackung des Bioreaktorbehälters 1 bzw. dessen Behälteröffnungen 9a, 9b, 9c. Der Sterilitätszustand innerhalb der Außenschicht 7 des Bioreaktorbehälters 1 und gegebenenfalls der Sterilitätszustand der durch die Außenschicht 7 eingeschlossenen Komponenten 17 kann mittels eines Sterilitätsindikators 19 dokumentiert werden. Beispielsweise kann der Sterilitätsindikator 19 anzeigen, z.B. durch Verfärbung, ob der Bioreaktorbehälter 1 bei einer Sterilisation mittels Gamma-Strahlung eine ausreichende Strahlendosis empfangen hat.

Beispielsweise durch ein Aufreißen der Außenschicht 7 an einer Trenngrenze 21 kann der Bioreaktorbehälter in einen zweiten Zustand überführt werden, in welchem die Behälteröffnung 9a, 9b, 9c bzw. die daran angeschlossenen bzw. damit verbundenen Komponenten 17 frei zugänglich sind. Die Außenschicht 7 kann hierbei derart aufgetrennt werden, daß die Außenschicht 7 zumindest bereichsweise mit der Innenschicht 5 verbunden bleibt. Ein Teil der Außenschicht kann entweder entfernt werden oder wird derart aufgetrennt, daß dieser Teil umgefaltet bzw. umgestülpt werden kann, um einen Zugang zu den Behälteröffnungen 9a, 9b, 9c bzw. den daran angeschlossenen Komponenten 17 zu erhalten.

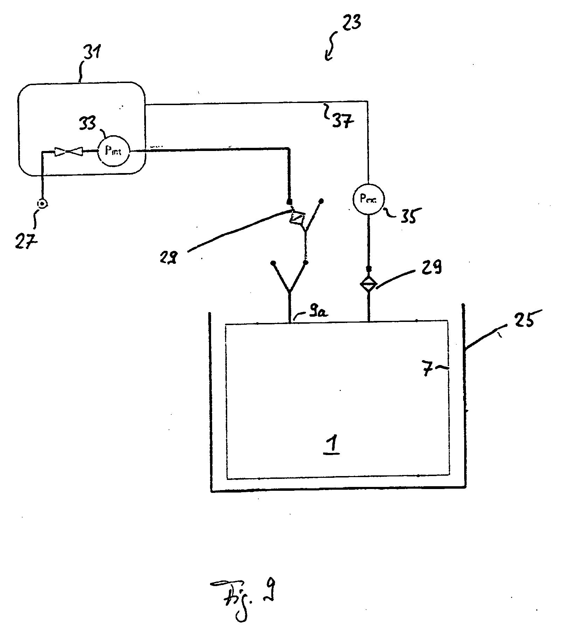

Die **Figur 9** zeigt eine schematische Ansicht einer Anordnung 23 zum Prüfen der Integrität des Bioreaktorbehälters 1. Zum Prüfen wird der Bioreaktorbehälter 1 in einer Bioreaktorbehälteraufnahme 25 einer Bioreaktorvorrichtung angeordnet. Die zumindest eine Behälteröffnung 9a des Bioreaktorbehälters 1 wird mit einer Fluidquelle 27 fluidisch verbunden, beispielsweise über einen Sterilfilter 29, um das Innere des Bioreaktorbehälters 1 steril zu halten. Die Fluidquelle kann Teil einer Prüfvorrichtung 31, wie z.B. Sartocheck® 4plus der Sartorius Stedium Biotech GmbH, sein oder eine externe Fluidquelle 27 sein.

Der Bioreaktorbehälter 1 wird mit einem Fluid aus der Fluidquelle 27 zu einem Zeitpunkt T₁ mit einem vorbestimmten Überdruck P₁ von etwa 50 mbar bis etwa 100 mbar gefüllt, wobei die Außenschicht 7 des Bioreaktorbehälters 1 zumindest bereichsweise an eine Innenwandung der Bioreaktorbehälteraufnahme 25 anliegt.

Die Druckdifferenz P₂-P₁ zwischen einem Überdruck P₂ zu einem späteren Zeitpunkt T₂ und dem bestimmten Überdruck zu Zeitpunkt T₁ kann mittels eines internen Drucksensors 33 der Prüfvorrichtung 31 erfaßt werden. Alternativ oder zusätzlich kann der Druck innerhalb des Bioreaktorbehälters 1 mittels eines externen Drucksensors 35 erfaßt werden. Der externe Drucksensor 35 ist bevorzugt mit der Prüfvorrichtung 31 über eine Signalleitung 37 verbunden. Anhand der Druckdifferenz P₂-P₁ kann bestimmt werden, ob der Bioreaktorbehälter 1 dicht bzw. integer ist. Dazu muß die Druckdifferenz kleiner als ein vorbestimmter Betrag sein, im Idealfall gleich null. Vorteilhafterweise kann der Bioreaktorbehälter 1 während der Prüfung an der Innenwandung der Bioreaktorbehälteraufnahme 25 anliegen, wobei das Ergebnis des Integritätstests nicht beeinflußt wird, da das Abdichten von vorhandenen Lecks durch die Innenwandung aufgrund der Beschaffenheit der Außenschicht 7 vermieden wird.

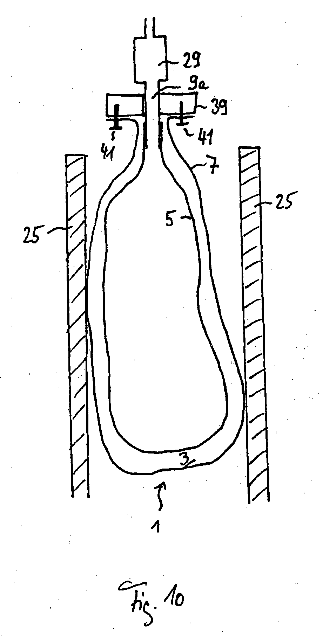

Die Figur 10 zeigt eine bevorzugte Ausführungsform eines Bioreaktorbehälters 1, welcher in einer Bioreaktoraufnahme 25 angeordnet ist. Der Bioreaktorbehälter 1 umfaßt eine Wandung 3, welche eine fluidundurchlässige Innenschicht 5 und eine fluiddurchlässige Außenschicht 7 aufweist. Die Wandung 3 kann vorzugsweise aus einer Folie bzw. aus einem Laminat mehrerer Folien bestehen. Mit anderen Worten kann die Wandung 3 im wesentlichen als flexibler Beutel ausgebildet sein, welcher in der Gestalt variieren kann. Die Innenschicht 5 ist aus einem flexiblen, nicht porösen Material ausgebildet, so daß durch die Innenschicht ein variables Innenvolumen umgeben ist. Das Innenvolumen des Bioreaktorbehälters 1 ist über eine Behälteröffnung 9a und einen Sterilfiler 29 mit der Umgebung oder mit weiteren Elementen, wie zum Beispiel Fluidleitungen, fluidisch verbindbar. Die Außenschicht 7 ist aus einem porösen, fluiddurchlässigen Material oder aus einem oberflächlich strukturierten Material ausgebildet, welches im wesentlichen beabstandet von der Innenschicht 5 angeordnet ist und mittels einer Befestigungseinrichtung 39 mit der Innenschicht 5 lösbar verbunden ist. Dazu kann die Außenschicht 7 mittels der Fixierelemente 41 an der Befestigungseinrichtung 39 fixiert werden. Nach der Prüfung können die Fixierelemente, beispielsweise Klemmen oder Schrauben, gelöst und die Außenschicht 7 entfernt werden.

### Bezugszeichenliste

- 1: Bioreaktorbehälter
- 3: Wandung
- 5: Innenschicht
- 7: Außenschicht
- 9a-d: Behälteröffnung
- 11: Lasche
- 13a-d: Durchtrittsöffnung
- 15: Schweißnaht
- 17: Komponente
- 19: Sterilitätsindikator
- 21: Trenngrenze
- 23: Anordnung zum Prüfen
- 25: Bioreaktorbehälteraufnahme
- 27: Fluidquelle
- 29: Sterilfilter
- 31: Prüfvorrichtung
- 33: interner Drucksensor
- 35: externer Drucksensor
- 37: Signalleitung
- L: Längsrichtung

## Patentansprüche

1. Bioreaktorbehälter (1) umfassend:
- eine zumindest bereichsweise flexible Wandung (3);
- zumindest eine Behälteröffnung (9a, 9b, 9c, 9d),
wobei die Wandung (3) des Bioreaktorbehälters (1) eine fluiddichte Innenschicht (5) aufweist, und
wobei die Wandung (3) des Bioreaktorbehälters (1) eine zumindest bereichsweise fluiddurchlässige Außenschicht (7) aufweist.

2. Bioreaktorbehälter (1) nach Anspruch 1, wobei die fluiddurchlässige Außenschicht (7) ein gewebtes Textil, ein nicht gewebtes Textil und/oder einen Schaumstoff umfaßt.

3. Bioreaktorbehälter (1) nach einem der vorigen Ansprüche, wobei die fluiddurchlässige Außenschicht (7) eine richtungsabhängige Fluiddurchlässigkeit aufweist.

4. Bioreaktorbehälter (1) nach einem vorigen Ansprüche, wobei die fluiddurchlässige Außenschicht (7) oder die strukturierte Außenschicht (5) mit der fluidundurchlässigen Innenschicht (5) lösbar oder unlösbar verbunden ist
und/oder
wobei die Außenschicht (7) als sterile Barriere ausgebildet ist.

5. Bioreaktorbehälter (1) nach einem der vorigen Ansprüche, wobei die zumindest eine Behälteröffnung (9a, 9b, 9c, 9d) des Bioreaktorbehälters (1) in einem ersten Zustand durch die Außenschicht (7) umhüllt ist, wobei die zumindest eine Behälteröffnung (9a, 9b, 9c, 9d) in einem zweiten Zustand frei zugänglich ist und wobei der Bioreaktorbehälter (1) durch ein bereichsweises Aufreißen und/oder Entfernen der Außenschicht (7) von dem ersten Zustand in den zweiten Zustand überführbar ist.

6. Verfahren zum Prüfen der Integrität eines Bioreaktorbehälters (1) mit den Schritten:
- Bereitstellen eines Bioreaktorbehälters (1) gemäß einem der vorigen Ansprüche;
- Bereitstellen einer Bioreaktorvorrichtung mit einer Bioreaktorbehälteraufnahme (25);
- Anordnen des Bioreaktorbehälters (1) zumindest teilweise in der Bioreaktorbehälteraufnahme (25);
- Verbinden der zumindest einen Behälteröffnung (9a, 9b, 9c, 9d) des Bioreaktorbehälters mit einer Fluidquelle (27);
- Füllen des Bioreaktorbehälters (1) mit einem Fluid aus der Fluidquelle (27), um zu einem ersten Zeitpunkt T₁ einen vorbestimmten Überdruck P₁ in dem Bioreaktorbehälter (1) zu erzeugen, wobei sich die Außenschicht (7) des Bioreaktorbehälters (1) zumindest bereichsweise an eine Innenwandung der Bioreaktorbehälteraufnahme (25) anlegt;
- Diskriminieren, ob der Bioreaktorbehälter (1) hinreichend dicht ist.

## Claims

1. A bioreactor container (1), comprising:
- an at least in some areas flexible wall (3),
- at least one container opening (9a, 9b, 9c, 9d),
the wall (3) of the bioreactor container (1) having a fluid-tight inner layer (5), and
the wall (3) of the bioreactor container (1) having an at least in some areas fluid- permeable outer layer (7).

2. A bioreactor container (1) according to claim 1, the fluid-permeable outer layer (7) comprising a woven fabric, a non-woven fabric and/or a foam.

3. A bioreactor container (1) according to one of the previous claims, the fluid-permeable outer layer (7) having a directional fluid permeability.

4. A bioreactor container (1) according to one of the previous claims, the fluid-permeable outer layer (7) or the structured outer layer (5) being releasably or non-releasably connected with the fluid-tight inner layer (5) and/or
the outer layer (7) being formed as a sterile barrier.

5. A bioreactor container (1) according to one of the previous claims, the at least one container opening (9a, 9b, 9c, 9d) of the bioreactor container (1) in a first state being enclosed by the outer layer (7), the at least one container opening (9a, 9b, 9c, 9d) in a second state being freely accessible and the bioreactor container (1) being transferable from the first state to the second state by means of tearing open and/or removing the outer layer (7) at least in some areas.

6. Method for verifying the integrity of a bioreactor container (1) with the following steps:
- providing a bioreactor container (1) according to one of the previous claims;
- providing a bioreactor device with a bioreactor container receptacle (25);
- arranging the bioreactor container (1) at least party in the bioreactor container receptacle (25);
- connecting the at least one container opening (9a, 9b, 9c, 9d) of the bioreactor container with a fluid source (27);
- filling the bioreactor container (1) with a fluid from the fluid source (27) in order to create at a first point of time T₁ a predetermined positive pressure P₁ in the bioreactor container (1), the outer layer (7) of the bioreactor container (1) being placed at least in some areas at an inner wall of the bioreactor container receptacle (25);
- discriminating whether the bioreactor container (1) is sufficiently tight.

## Revendications

1. Conteneur de bioréacteur (1) comprenant :
- une paroi (3) flexible au moins par zones;
- au moins une ouverture de conteneur (9a, 9b, 9c, 9d).
sachant que la paroi (3) du conteneur de bioréacteur (1) présente une couche intérieure (5) étanche aux fluides, et
sachant que la paroi (3) du conteneur de bioréacteur (1) présente une couche extérieure (7) perméable aux fluides au moins par zones.

2. Conteneur de bioréacteur (1) d'après la revendication 1, sachant que la couche extérieure (7) perméable aux fluides comprend un textile tissé, un textile non-tissé et/ou un matériau alvéolaire.

3. Conteneur de bioréacteur (1) d'après une des revendications précédentes, sachant que la couche extérieure (7) perméable aux fluides présente une perméabilité aux fluides directionnelle.

4. Conteneur de bioréacteur (1) d'après une des revendications précédentes, sachant que la couche extérieure (7) perméable aux fluides ou la couche extérieure (5) structurée est raccordée de manière amovible ou inamovible à la couche intérieure (5) étanche aux fluides
et/ou
sachant que la couche extérieure (7) est réalisée en tant que barrière stérile.

5. Conteneur de bioréacteur (1) d'après une des revendications précédentes, sachant que la ou les ouvertures de conteneur (9a, 9b, 9c, 9d) du conteneur de bioréacteur (1) dans un premier état sont enrobées par la couche extérieure (7), sachant que la ou les ouvertures de conteneur (9a, 9b, 9c, 9d) dans un deuxième état sont librement accessibles et sachant que le conteneur de bioréacteur (1) peut être amené du premier état au deuxième état en déchirant et/ou éliminant par zones la couche extérieure (7).

6. Procédé de contrôle d'intégrité d'un conteneur de bioréacteur (1) avec les étapes suivantes :
- fournir un conteneur de bioréacteur (1) d'après une des revendications précédentes ;
- fournir un dispositif de bioréacteur avec un logement (25) de conteneur de bioréacteur ;
- disposer le conteneur de bioréacteur (1) au moins partiellement dans le logement (25) de conteneur de bioréacteur ;
- raccorder le ou les ouvertures de conteneur (9a, 9b, 9c, 9d) du conteneur de bioréacteur avec une source de fluide (27) ;
- remplir le conteneur de bioréacteur (1) avec un fluide de la source de fluide (27), pour produire à un premier moment T₁ une première surpression prédéterminée P₁ dans le conteneur de bioréacteur (1), sachant que la couche extérieure (7) du conteneur de bioréacteur (1) se place au moins par zones contre une paroi intérieure du logement (25) de conteneur de bioréacteur ;
- discriminer si le conteneur de bioréacteur (1) est suffisamment étanche.
